# DEMANDE DE BREVET EUROPEEN

(11) **EP 3 391 811 A1**
(43) Date de publication de la demande: **24.10.2018**
(21) Numéro de dépôt: 18167366.6
(22) Date de dépôt: 13.04.2018
(51) Int. Cl.: A61B 5/00, A61B 1/24

(54) **DISPOSITIF DE PRISE DE VUE DENTAIRE**

(30) Priorité: 19.04.2017 FR 1753392
(71) Demandeur: Dental Monitoring, 75008 Paris (FR)
(72) Inventeur: DEBRAUX, Laurent, 75020 Paris (FR); SALAH, Philippe, 93170 Bagnolet (FR); GHYSELINCK, Guillaume, 59169 Cantin (FR); PELLISSARD, Thomas, 92110 Clichy (FR)
(74) Mandataire: Nony

(57) **Abrégé**

Dispositif de prise de vue comportant :
- un support (12) ;
- un écarteur dentaire (14) fixé sur le support et définissant une ouverture d'écarteur (24) ;
- une mire colorimétrique et/ou une mire de translucidité ; et
- une source lumineuse orientée de manière à éclairer d'une part les dents du patient à travers l'ouverture d'écarteur et d'autre part ladite mire colorimétrique et/ou ladite mire de translucidité ;
- des moyens de fixation (18) d'un appareil d'acquisition d'images (19) sur le support dans une position dans laquelle l'appareil d'acquisition est orienté pour recevoir une image (I_{c}) de l'ouverture d'écarteur d'une part et de ladite mire colorimétrique et/ou de ladite mire de translucidité d'autre part.

## Description

### Domaine technique

La présente invention concerne un dispositif de prise de vue dentaire, en particulier pour la mise en oeuvre d'un procédé tel que décrit dans la demande internationale PCT/EP2015/074896.

### Etat de la technique

PCT/EP2015/074896 décrit un procédé permettant, à partir
- d'un modèle tridimensionnel des dents d'un patient réalisé avant le traitement, dit « modèle de référence initial », puis
- d'une simple image « actualisée » des dents prise pendant le traitement, par exemple d'une photographie prise par le patient,
d'évaluer avec précision le positionnement des dents au moment de l'acquisition de l'image actualisée.

Ce procédé consiste en un processus itératif selon lequel, à chaque itération, des modèles de dents du modèle de référence initial sont déplacés, puis des conditions optimales d'observation du modèle initial ainsi modifié (dit « modèle de référence à tester ») sont déterminées, les conditions optimales d'observation étant définies comme les conditions permettant d'observer le modèle de référence à tester de manière que la vue dudit modèle soit la plus proche possible de l'image actualisée. On teste une succession de « modèles de référence à tester », jusqu'à obtenir un niveau de correspondance maximal entre un modèle de référence à tester et l'image actualisée. Ce dernier modèle de référence à tester est alors considéré comme représentant les dents dans leur position au moment de l'acquisition de l'image actualisée. Idéalement, ce modèle, dit « modèle de référence actualisé », est un modèle de référence tridimensionnel numérique à partir duquel l'image actualisée aurait pu être prise si ce modèle avait été réel. En pratique, il représente effectivement, avec une grande précision, les dents dans leur position au moment de l'acquisition de l'image actualisée.

Généralement plusieurs images actualisées sont nécessaires pour évaluer le positionnement de plusieurs dents. Pour chaque image actualisée, le procédé doit être mis en oeuvre. Le procédé décrit dans la demande internationale PCT/EP2015/074896 peut donc être long à mettre en oeuvre.

Par ailleurs, les images actualisées peuvent être utilisées pour détecter des évolutions de l'aspect des dents ou des tissus mous comme les gencives, et en particulier de leur couleur et de leur translucidité. La comparaison de différentes photos ne fournit cependant pas toujours des résultats satisfaisants.

Un objectif de la présente invention est de répondre, au moins partiellement, à ces problèmes.

### Résumé de l'invention

L'invention propose un dispositif de prise de vue comportant :
- un support ;
- un écarteur dentaire fixé sur le support et définissant une ouverture d'écarteur ;
- des moyens de fixation d'un appareil d'acquisition d'images sur le support dans une position dans laquelle l'appareil d'acquisition est orienté pour recevoir une image de l'ouverture d'écarteur.

Suivant un premier aspect principal, le dispositif comporte un miroir fixé sur le support et les moyens de fixation sont configurés pour une fixation de l'appareil d'acquisition d'images sur le support dans une position dans laquelle l'appareil d'acquisition est orienté pour recevoir une image composée comportant une image directe de l'ouverture d'écarteur et une image de l'ouverture d'écarteur réfléchie par le miroir.

Dans une position de service dans laquelle l'écarteur est disposé sur la bouche du patient et l'appareil d'acquisition d'images est fixé sur le support par lesdits moyens de fixation, ledit appareil d'acquisition voit ainsi une image composée comportant une image directe des dents observée directement à travers l'ouverture d'écarteur et une image réfléchie des dents renvoyée par le miroir.

Comme cela apparaîtra plus clairement dans la suite de la description, un dispositif selon le premier aspect principal de l'invention permet ainsi d'acquérir simultanément plusieurs images des dents, observées selon des angles différents. La procédure d'acquisition en est accélérée.

En outre, la détermination des conditions d'acquisition de l'image directe, ou de l'image réfléchie, permet la détermination des conditions d'acquisition de l'image réfléchie, ou de l'image directe respectivement. Il suffit en effet de connaître l'orientation du miroir et sa position par rapport à l'écarteur et à l'appareil d'acquisition pour déterminer le positionnement dans l'espace de l'appareil d'acquisition qui aurait permis, en l'absence du miroir, d'observer l'image réfléchie. La mise en oeuvre d'un procédé tel que celui dans la demande internationale PCT/EP2015/074896 en est dès lors considérablement accélérée.

En effet, typiquement, le patient prend une photo de ses dents depuis la droite, une photo de ses dents depuis la gauche et une photo de ses dents vues de face. Mais le logiciel mettant en oeuvre le procédé décrit dans la demande internationale PCT/EP2015/074896 ignore la position de l'appareil d'acquisition à ces différentes étapes et ne peut la déduire d'un traitement des autres images. Il doit donc, à chaque fois, la retrouver pour définir les conditions optimales d'observation.

Suivant un deuxième aspect principal, le dispositif comporte
- une mire colorimétrique et/ou une mire de translucidité, de préférence une mire colorimétrique et une mire de translucidité, fixée(s) de préférence sur le support ; et
- une source lumineuse orientée de manière à éclairer d'une part les dents du patient à travers l'ouverture d'écarteur et d'autre part ladite mire colorimétrique et/ou ladite mire de translucidité,
lesdits moyens de fixation de l'appareil d'acquisition étant configurés pour immobiliser l'appareil d'acquisition dans une position dans laquelle il est orienté pour recevoir une image de l'ouverture d'écarteur d'une part et de ladite mire colorimétrique et/ou de ladite mire de translucidité d'autre part.

Comme cela apparaîtra plus clairement dans la suite de la description, un dispositif selon le deuxième aspect principal de l'invention permet ainsi de déterminer avec précision la couleur et la translucidité des dents et/ou des tissus mous comme les gencives. En particulier, l'éclairage peut être contrôlé de manière à limiter les perturbations par l'environnement lumineux du dispositif. Différentes images prises à des instants différents peuvent donc être comparées avec une bonne précision.

Bien entendu, les caractéristiques des deux aspects principaux de l'invention peuvent être combinées.

Quel que soit l'aspect principal considéré, un dispositif selon l'invention présente de préférence une ou plusieurs des caractéristiques optionnelles suivantes :
- le support définit une chambre débouchant vers l'extérieur par l'ouverture d'écarteur et par une ouverture d'acquisition à travers laquelle l'appareil d'acquisition fixé par lesdits moyens de fixation reçoit ladite image composée ;
- le support est télescopique de manière que la distance entre les ouvertures d'écarteur et d'acquisition soit variable ;
- le dispositif comporte au moins deux dits miroirs orientés perpendiculairement l'un à l'autre et renvoyant chacun une image réfléchie de l'ouverture d'écarteur, chacune desdites images réfléchies étant représentée sur ladite image composée ;
- les moyens de fixation de l'appareil d'acquisition sur le support sont désactivables ;
- les moyens de fixation de l'appareil d'acquisition et/ou de l'écarteur sur le support sont choisis dans le groupe constitué par un clips, une bande auto-agrippante, des mâchoires de serrage, une vis, un aimant, un capot et une forme complémentaire entre le support et l'appareil d'acquisition ;
- le dispositif comporte des moyens de fixation de l'écarteur sur le support désactivables, l'écarteur pouvant ainsi être fixé de manière amovible sur le support ;
- l'écarteur comporte des pattes qui, dans une position montée de l'écarteur sur le support, sont insérées dans des logements profilés respectifs du support, chaque logement présentant une section transversale de forme générale en U, l'ouverture du U débouchant de préférence vers le haut ou vers le bas dans une position de service dans laquelle l'écarteur est disposé sur la bouche d'un patient maintenant sa tête verticale ;
- dans ladite position montée, l'écarteur est maintenu en flexion, en appui élastique sur le support ;
- le support comporte un ou plusieurs, de préférence deux crochets configurés pour recevoir l'écarteur dans ladite position montée ;
- la source lumineuse est configurée de manière à projeter un repère vers l'ouverture d'écarteur ;
- le dispositif comporte un module de contrôle configuré pour contrôler les propriétés du rayonnement émis par la source lumineuse, de préférence en fonction du rayonnement lumineux reçu par l'ouverture d'écarteur ;
- le module de contrôle est configuré pour commander la source lumineuse pour que plus de 50%, plus de 70%, plus de 90%, voire sensiblement 100% de l'intensité du rayonnement reçu par l'ouverture d'écarteur proviennent de la source lumineuse ;
- le support présente la forme d'un boîtier ne débouchant vers l'extérieur sensiblement que par l'ouverture d'écarteur et par une ouverture d'acquisition à travers laquelle l'appareil d'acquisition fixé sur le support reçoit au moins une image de l'ouverture d'écarteur ;
- le dispositif comporte un module de traitement dans lequel sont enregistrées les propriétés de couleur et de translucidité réelles des mires colorimétrique et de translucidité, respectivement, le module de traitement comprenant des instructions de code de programme pour corriger une image représentant lesdites mires de manière que les représentations desdites mires sur l'image présentent lesdites propriétés de couleur et de translucidité.

L'invention concerne également :
- un programme d'ordinateur, et en particulier un applicatif spécialisé pour téléphone mobile, comprenant des instructions de code de programme pour
   - guider un opérateur lors d'un réglage de la géométrie du support et/ou d'une orientation du miroir et/ou d'un positionnement de la bouche du patient sur l'écarteur, et/ou
   - pour commander un ou plusieurs actionneurs aptes à modifier ladite géométrie, notamment modifier la longueur du support, et/ou ladite orientation du miroir,
- un support informatique sur lequel est enregistré un tel programme, par exemple une mémoire ou un CD-ROM, et
- un appareil personnel, en particulier un téléphone mobile ou une tablette, dans lequel est chargé un tel programme.

L'invention concerne également un kit de prise de vue comportant :
- un dispositif de prise de vue selon l'invention ; et
- un appareil d'acquisition d'images, de préférence fixé sur le dispositif de manière à acquérir lesdites images, et en particulier ladite image composée.

Un kit selon l'invention présente de préférence une ou plusieurs des caractéristiques optionnelles suivantes :
- l'appareil d'acquisition est un téléphone mobile ;
- l'appareil d'acquisition d'images comporte un programme d'ordinateur selon l'invention ;
- l'appareil d'acquisition d'images est configuré pour prendre successivement plusieurs photos, avec des distances focales différentes, en conséquence d'un unique déclenchement.

### Définitions

Par « patient », on entend toute personne pour laquelle un dispositif selon l'invention peut être mis en oeuvre, que cette personne soit malade ou non, ou que cette personne soit en cours de traitement ou non. Un dispositif selon l'invention peut être utilisé pour un autre animal qu'un être humain.

On appelle "téléphone mobile" un appareil de moins de 500 g, doté d'un capteur lui permettant de capturer des images, capable d'échanger des données avec un autre appareil éloigné de plus de 500 km du téléphone mobile, et capable d'afficher lesdites données, et notamment lesdites images.

Les « conditions d'acquisition » d'une image des dents précisent la position et l'orientation dans l'espace d'un appareil d'acquisition d'images relativement aux dents du patient, et de préférence la calibration de cet appareil d'acquisition d'images, pour acquérir à l'instant de ladite acquisition, par observation directe, ladite image. Les conditions d'acquisition d'une image réfléchie précisent donc la position et l'orientation dans l'espace que devrait prendre l'appareil d'acquisition d'images, en l'absence du miroir, pour acquérir l'image réfléchie.

La « calibration » d'un appareil d'acquisition est constituée par l'ensemble des valeurs des paramètres de calibration. Un paramètre de calibration est un paramètre intrinsèque à l'appareil d'acquisition (à la différence de sa position et de son orientation) dont la valeur influence l'image acquise. Par exemple, l'ouverture de diaphragme est un paramètre de calibration qui modifie la profondeur de champ. Le temps d'exposition est un paramètre de calibration qui modifie la luminosité (ou « l'exposition ») de l'image. La distance focale est un paramètre de calibration qui modifie l'angle de vue, c'est-à-dire le degré de « zoom ». La « sensibilité » est un paramètre de calibration qui modifie la réaction du capteur d'un appareil d'acquisition numérique à la lumière incidente.

De préférence, les paramètres de calibration sont choisis dans le groupe formé par l'ouverture de diaphragme, le temps d'exposition, la distance focale et la sensibilité.

Par "image", on entend une image en deux dimensions, comme une photographie. Une image est formée de pixels.

« Fixer » signifie « solidariser de manière rigide ou de manière à n'autoriser qu'un déplacement guidé ». « Fixer » ne signifie pas nécessairement « solidariser de manière définitive ».

"Comprendre", "comporter" et "présenter" doivent être interprétés de manière large et non limitative, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente, en perspective, un dispositif selon l'invention, observé par transparence du côté de l'écarteur ;
- la figure 2 représente, en perspective, un dispositif selon l'invention représenté du côté de l'ouverture d'acquisition ;
- la figure 3 représente un exemple d'image composée ;
- les figures 4a, 4b et 4c représentent le dispositif de la figure 1 vu de dessus, vu du côté de l'ouverture d'acquisition et vu du côté de l'ouverture d'écarteur, respectivement ;
- les figures 5a et 5b représentent le dispositif de la figure 2 dans des positions rétractée et déployée du support, respectivement ; et
- les figures 6a, 6b et 6c représentent en perspective (figures 6a et 6b) et vue de dessus (figure 6c) la partie du support d'un dispositif selon l'invention sur laquelle un écarteur peut être monté, comme représenté sur la figure 6b.

Dans les différentes figures, les organes identiques ou analogues ont été désignés avec des références identiques.

### Description détaillée

### Dispositif

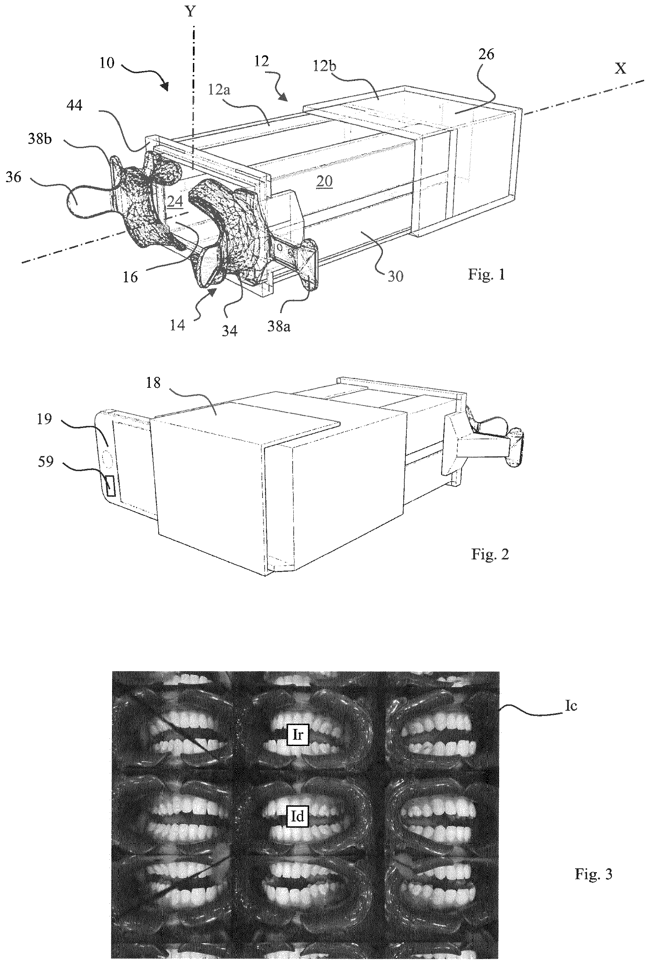

Le dispositif de prise de vue 10 représenté sur la figure 1 comporte un support 12, sous la forme d'un boîtier de préférence télescopique, un écarteur dentaire 14, au moins un miroir 16 et des moyens de fixation 18 d'un appareil d'acquisition d'images 19, représentés sur la figure 2.

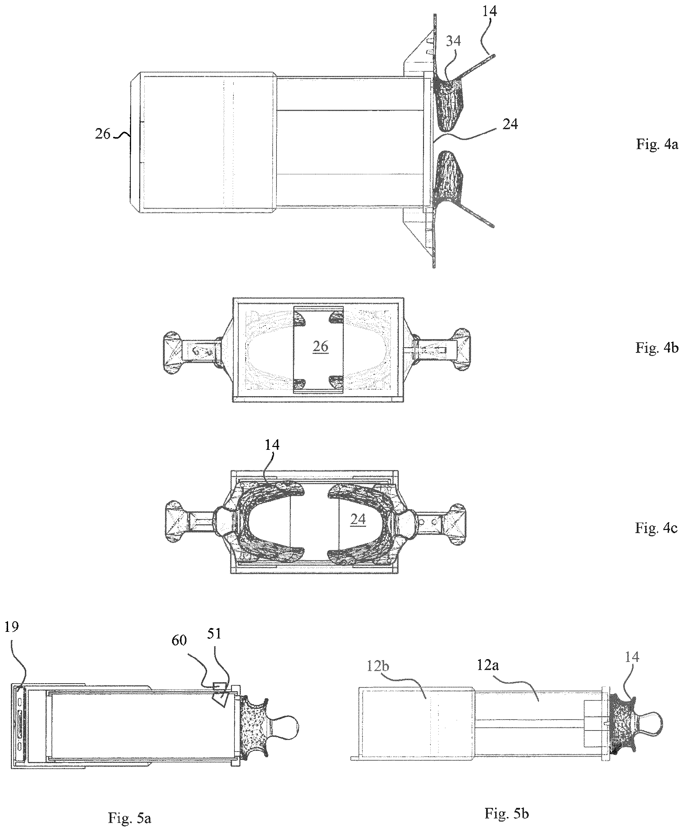

Le support 12 comporte une partie mâle 12a et une partie femelle 12b montées coulissantes l'une dans l'autre, suivant un axe d'écarteur X, entre des positions rétractée (figure 5a) et déployée (figure 5b). Le support 12 définit une chambre 20 dont la longueur selon l'axe X dépend de la position relative des parties mâle et femelle du support 12.

Dans le mode de réalisation représenté, la chambre 20 débouche vers l'extérieur sur deux faces d'extrémité opposées du support 12, par une ouverture d'écarteur 24 et une ouverture d'acquisition 26, respectivement. La paroi latérale 30 du support 12, qui s'étend entre les deux faces d'extrémité, est sensiblement cylindrique d'axe X, de section rectangulaire.

Le boîtier ainsi constitué peut être par exemple en plastique ou en carton.

Les moyens de fixation 18 sont configurés de manière que l'appareil d'acquisition d'images puisse être fixé dans une position d'acquisition dans laquelle son objectif fait face à l'ouverture d'acquisition 26, voire obture l'ouverture d'acquisition 26.

Les moyens de fixation 18 de l'appareil d'acquisition sur le support 12 peuvent être quelconques. De préférence, ils permettent une fixation rigide. De préférence, ils sont désactivables, c'est-à-dire que l'utilisateur peut désolidariser l'appareil d'acquisition du support dès qu'il le souhaite.

De préférence, les moyens de fixation de l'appareil d'acquisition sont choisis dans le groupe constitué par des moyens de clipsage, des bandes auto-agrippantes de type Velcro®, des mâchoires de serrage, des vis, des aimants, et une complémentarité de forme entre le support et l'appareil d'acquisition. Dans le mode de réalisation représenté sur la figure 2, les moyens de fixation 18 sont constitués par un capot qui peut être serré contre le support 12.

Dans un mode de réalisation, les moyens de fixation 18 sont adaptés pour la fixation d'un appareil photo conventionnel, par exemple de type reflex.

L'écarteur 14 peut présenter les caractéristiques des écarteurs conventionnels. Il comporte classiquement un rebord 34 s'étendant autour de l'ouverture d'écarteur 24 et agencé de manière que les lèvres du patient puissent y reposer en laissant apparaître les dents du patient à travers ladite ouverture d'écarteur. Dans le mode de réalisation représenté, l'écarteur 14 comporte également des oreilles 36 agencées de manière à écarter les joues des dents et des pattes droite 38a et gauche 38b, sensiblement perpendiculaires à l'axe X, facilitant sa manipulation.

Le rebord 34 présente la forme d'une goulotte apte à retenir les lèvres du patient.

L'écarteur 14 est de préférence en un matériau biocompatible, par exemple en matière plastique.

L'écarteur 14 peut être venu de matière avec le support 12 ou être fixé, de préférence rigidement, sur le support 12 par tout moyen.

De préférence, l'écarteur est amovible, c'est-à-dire qu'il peut être monté et démonté du support par l'opérateur. Avantageusement, le même support peut donc servir pour plusieurs écarteurs, et en particulier pour plusieurs écarteurs de tailles différentes.

Les moyens de fixation de l'écarteur sur le support peuvent être par exemple des moyens de clipsage, des bandes auto-agrippantes de type Velcro®, des mâchoires de serrage, des vis, des aimants, et une complémentarité de forme entre le support et l'écarteur.

Dans un mode de réalisation, l'écarteur 14 est fixé au moyen d'une insertion d'une des pattes droite 38a et gauche 38b dans un logement du support, puis d'un clipsage de l'autre patte sur le support.

Dans un mode de réalisation, l'écarteur 14 est fixé au moyen d'un clipsage des pattes dans des logements du support respectifs.

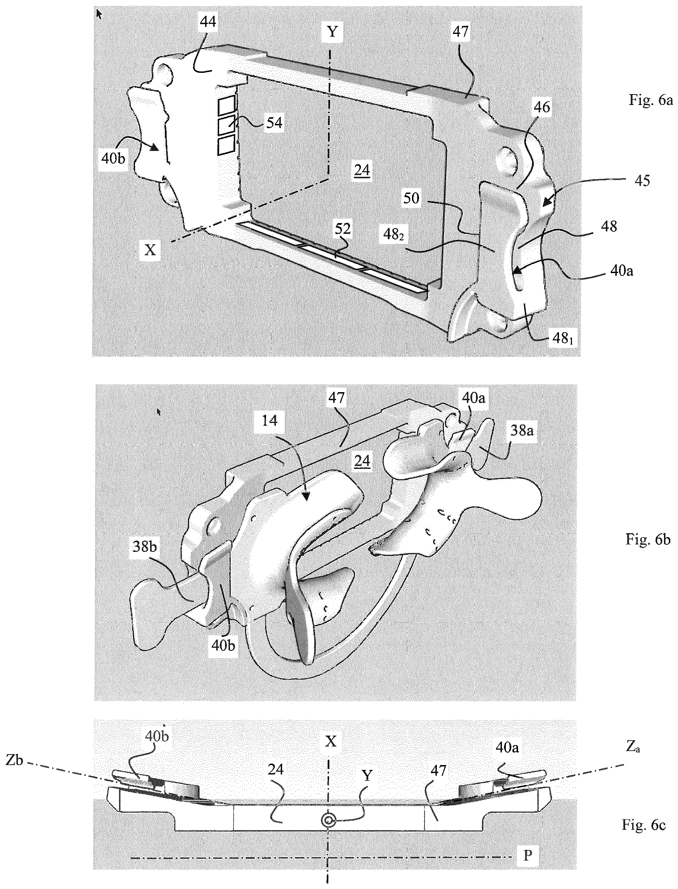

Dans un mode de réalisation préféré, illustré par la figure 6, les moyens de fixation de l'écarteur sur le support comportent un crochet sur lequel l'écarteur 14 peut être accroché. Les moyens de fixation de l'écarteur sur le support comportent de préférence un crochet droit 40a et un crochet gauche 40b agencés pour recevoir les pattes transversales droite 38a et gauche 38b de l'écarteur, respectivement. Les crochets droit et gauche étant similaires, seul un crochet est décrit en détail ci-après.

De préférence, ledit crochet fait saillie de la face arrière 44 du support, sensiblement perpendiculaire à l'axe X, comme représenté sur la figure 1. La face arrière 44 est de préférence définie par une plaque 47 (figure 6a) fixée sur un chant de la paroi latérale 30 du support 12.

De préférence, le crochet présente la forme d'une cornière 45. La cornière comporte de préférence des première et deuxième ailes perpendiculaires l'une à l'autre. La première aile 48₁, fixée sur la face arrière 44 du support, est de préférence sensiblement parallèle à l'axe X et la deuxième aile 48₂ est de préférence sensiblement perpendiculaire à l'axe X et, de préférence, s'étend vers le haut depuis la première aile. Avec la face arrière 44 du support, la cornière 45 définit un logement profilé en U dont l'ouverture principale 46, orientée vers le haut, est dimensionnée pour recevoir une patte de l'écarteur. De préférence encore, le logement profilé débouche, à ses deux extrémités droite et gauche, par des ouvertures droite 48 et gauche 50 à travers lesquelles une patte de l'écarteur peut être glissée dans le crochet, c'est-à-dire entre la face arrière 44 du support et la deuxième aile 48₂ de la cornière 45, jusqu'à entrer en butée avec la première aile 48₁.

De préférence, les crochets sont configurés de manière que l'écarteur ne puisse y être inséré qu'en force, de préférence par ouverture élastique des crochets.

De préférence, les crochets sont configurés de manière que l'écarteur ne puisse y être inséré sans être déformé, de préférence par flexion autour d'un axe Y sensiblement perpendiculaire à l'axe X, de préférence sensiblement vertical dans la position de service.

De préférence encore, dans la position montée de l'écarteur sur le support, les moyens de fixation maintiennent l'écarteur fléchi autour de l'axe Y.

Dans le mode de réalisation de la figure 6 (figure 6c), les axes Zₐ et Z_{b} des cornières des crochets droit et gauche sont ainsi inclinés par rapport à un plan transversal P perpendiculaire à l'axe X, alors que les pattes droite et gauche de l'écarteur sont sensiblement coplanaires lorsque l'écarteur est au repos, désolidarisé du support. La largeur des ouvertures principales 46 des crochets droit et gauche est sensiblement identique à l'épaisseur des pattes droite et gauche de l'écarteur, respectivement, ce qui oblige l'opérateur à fléchir l'écarteur autour de l'axe Y pour insérer ces pattes dans ces crochets. La forme des crochets empêche ensuite un retour de l'écarteur dans sa position de repos.

L'appui élastique de l'écarteur sur le support ainsi obtenu favorise avantageusement son maintien en position.

Dans un mode de réalisation, le dispositif comporte encore un appareil d'acquisition d'images 19.

L'appareil d'acquisition d'images fournit de préférence des images en couleurs, et/ou des images infrarouges. Les images infrarouges permettent avantageusement de faire apparaître les dents avec un excellent contraste.

De préférence, l'appareil d'acquisition d'images est un appareil personnel couramment disponible dans le commerce, par exemple un téléphone mobile, un appareil photo dit « connecté », une montre dite « intelligente », ou « smartwatch », ou une tablette ou un ordinateur personnel, fixe ou portable, comportant un système d'acquisition d'images, comme une webcam ou un appareil photo, de préférence un appareil photo numérique. Il pèse de préférence moins de 3 kg, moins de 2 kg, moins de 1 kg, moins de 500 g, de préférence moins de 300 g.

L'appareil d'acquisition d'images peut être intégré dans le support 12 ou être fixé provisoirement, grâce à des moyens de fixation 18 désactivables, sur le support 12.

Dans un mode de réalisation préféré, l'appareil d'acquisition d'images comporte un applicatif configuré pour guider l'opérateur, notamment pour qu'il ajuste de manière appropriée la longueur du support 12. De préférence, l'applicatif guide également le patient afin qu'il positionne correctement sa bouche sur l'écarteur 14.

Dans un mode de réalisation, le support 12 est pourvu d'une fenêtre à travers laquelle la lumière de l'environnement peut pénétrer à l'intérieur de la chambre 20 pour éclairer les dents.

Dans un mode de réalisation préféré, le dispositif comporte une source lumineuse 51 orientée vers l'ouverture d'écarteur 24 (fig. 5a) de manière à éclairer les dents du patient à travers l'ouverture d'écarteur 24. La source lumineuse 51 peut en particulier émettre une lumière blanche, une lumière monochrome, un rayonnement infrarouge ou, de préférence, un rayonnement ultraviolet.

La source lumineuse 51 peut être un flash.

Dans un mode de réalisation, la source lumineuse 51 est configurée de manière à projeter sur les dents, à travers l'ouverture d'écarteur 24, un repère, de préférence une grille laser. Avantageusement, la représentation d'un repère sur les images facilite la détermination de la forme des dents. Une première estimation de la forme des dents est ainsi possible sans mettre en oeuvre le procédé décrit dans PCT/EP2015/074896.

Dans un mode de réalisation, un filetage est ménagé sur la paroi latérale 30 du boîtier, de préférence sur la paroi latérale de la partie femelle 12b du support. Le filetage est de préférence conformé de manière à autoriser la fixation du support sur un trépied pour appareil photographique.

Dans un mode de réalisation, le dispositif comporte encore un capot d'acquisition et un capot d'écarteur, conformés pour sélectivement obturer l'ouverture d'acquisition et l'ouverture d'écarteur pour faciliter le stockage du dispositif. De préférence, le capot d'écarteur est conformé pour sélectivement obturer l'ouverture d'écarteur après démontage de l'écarteur.

De préférence, le dispositif comporte encore un module de traitement 59, en particulier un applicatif de l'appareil d'acquisition d'images 19 ou un logiciel chargé dans un ordinateur ou une tablette.

**Dans le premier mode de réalisation principal de l'invention**, le dispositif comporte un miroir 16.

Le miroir 16 est fixé sur la face intérieure de la paroi latérale 30.

Le miroir 16 est de préférence plan.

Le nombre et la forme des miroirs ne sont pas limitatifs. En particulier, le miroir peut être de forme rectangulaire, sphérique, ovale, octogonale ou hexagonale.

Dans le mode de réalisation représenté, des miroirs 16 tapissent toute la face intérieure de la paroi latérale de la chambre 20, au moins dans la partie femelle 12b du support 12 et, de préférence, également dans la partie mâle 12a du support.

Dans un mode de réalisation, le dispositif comporte quatre miroirs disposés chacun sur une des quatre faces de la paroi latérale du support, et en particulier de la partie femelle 12b et, de préférence, de la partie mâle 12a du support. Dans un mode de réalisation préféré, chaque miroir couvre entièrement la face du support 12 sur laquelle il s'étend.

La longueur d'un miroir peut être supérieure à 3 cm, supérieure à 5 cm et/ou inférieure à 30 cm, inférieure à 20 cm, inférieure à 15 cm, ou inférieure à 10 cm. La largeur d'un miroir peut être supérieure à 2 cm, supérieur à 3 cm et/ou inférieure à 10 cm ou inférieure à 8 cm.

Dans le mode de réalisation représenté, les miroirs sont fixés sur le support, de préférence de manière définitive, par exemple au moyen d'une colle.

Le miroir 16, de préférence chaque miroir 16, s'étend de préférence parallèlement à l'axe X. Dans le mode de réalisation représenté, les miroirs 16 s'étendent perpendiculairement les uns aux autres, deux à deux.

La présence simultanée d'un miroir et d'une source lumineuse 51 est particulièrement avantageuse puisqu'elle permet d'acquérir des images des dents faisant apparaitre simultanément le reflet de la source lumineuse 51 sur les dents, mais également le reflet de la source lumineuse fictive obtenue par la réflexion de la source lumineuse 51 par le miroir. L'analyse de la position relative de ces reflets dans les images permet avantageusement de déterminer grossièrement l'orientation de la surface des dents les renvoyant, ainsi que la position de cette surface par rapport à l'appareil d'acquisition.

**Dans le deuxième mode de réalisation principal de l'invention**, le dispositif comporte une mire colorimétrique 52 et/ou une mire de translucidité 54 est fixée sur le support, de préférence dans la chambre 20.

Le nombre et la forme des mires ne sont pas limitatifs. Dans un mode de réalisation de la figure 6a, le support porte par exemple trois mires colorimétriques 52 et trois mires de translucidité 54.

Avantageusement, les mires colorimétrique(s) 52 et de translucidité 54 permettent, pour chaque image, de corriger les erreurs de teinte propres à chaque appareil d'acquisition d'images.

Les mires colorimétrique(s) 52 et de translucidité 54 permettent également avantageusement de déterminer les couleurs et la translucidité exactes des dents ou des gencives, ce qui permet de détecter toute évolution de ces propriétés.

De préférence, les mires colorimétrique(s) 52 et de translucidité 54 sont fixées à proximité de l'ouverture d'écarteur, de préférence à moins de 5 cm, moins de 3 cm, moins de 1 cm de l'ouverture d'écarteur. De préférence, les mires colorimétrique(s) 52 et de translucidité 54 sont fixées sensiblement dans le plan de l'ouverture d'écarteur, comme représenté sur la figure 6a.

De préférence, le module de contrôle 60 contrôle les propriétés du rayonnement émis par la source lumineuse 51, de préférence en fonction du rayonnement lumineux reçu par l'ouverture d'écarteur. Un capteur de lumière peut être prévu, à proximité de l'ouverture d'écarteur, pour évaluer le rayonnement lumineux reçu par ladite ouverture d'écarteur.

Dans un mode de réalisation, le module de contrôle 60 contrôle la puissance de la source lumineuse 51 pour que plus de 50%, plus de 70%, plus de 90%, voire sensiblement 100% de l'intensité du rayonnement reçu par l'ouverture d'écarteur proviennent de la source lumineuse 51. La mise en oeuvre d'un support présentant la forme d'un boîtier ne débouchant vers l'extérieur sensiblement que par les ouvertures d'écarteur et d'acquisition limite l'influence de l'environnement lumineux extérieur au boîtier et permet donc avantageusement de limiter la puissance de la source lumineuse 51.

Un tel boîtier permet également une acquisition des images plus intime.

### Fonctionnement

Le fonctionnement du dispositif découle directement de la description qui précède.

L'opérateur vient d'abord fixer l'appareil d'acquisition d'images 19 sur le support 12 au moyen des moyens de fixation 18, de manière que l'appareil d'acquisition d'images 19 puisse observer à la fois l'image directe et l'image réfléchie.

L'opérateur règle alors la position, selon l'axe X, de la partie femelle par rapport à la partie mâle 12a, en fonction de l'appareil d'acquisition d'images 19 et de son réglage. Dans un mode de réalisation, une échelle est disposée sur la partie mâle 12a du support. De préférence, cette échelle fournit des indications facilitant le réglage de la longueur, selon l'axe X, du support 12, par exemple en portant une marque pour chaque type d'appareil d'acquisition d'images.

De préférence, l'opérateur allume la source lumineuse 51 de manière à éclairer les dents et projeter sur les dents le repère, et en particulier une grille laser.

L'opérateur fixe également l'écarteur sur le support. Dans un mode de réalisation représenté, il insère une première des pattes droite et gauche derrière un premier des crochets droit et gauche, respectivement, puis plie légèrement l'écarteur pour autoriser l'insertion de la deuxième patte derrière le deuxième crochet. Lorsque l'opérateur relâche son action, l'écarteur tend à reprendre sa forme initiale, mais ce retour vers la forme initiale est entravé par les crochets. La pression de l'écarteur sur le support ainsi obtenue garantit le maintien en position de l'écarteur derrière les crochets.

Le patient, qui peut être également l'opérateur, dispose alors ses lèvres dans les goulottes définies par le rebord 34 de l'écarteur. Comme représenté sur la figure 3, les dents du patient sont alors bien dégagées.

Le dispositif est alors dans une position de service dans laquelle l'appareil d'acquisition d'images 19 voit une image composée I_{c} du type de celle représentée sur la figure 3. Sur cette figure, l'image composée comporte une image directe I_{d} et 8 images réfléchies Iᵣ réfléchies par les différents miroirs 16 disposés dans la chambre 20.

En appuyant sur le déclencheur de l'appareil d'acquisition, l'opérateur acquiert l'image composée I_{c}. Avantageusement, il acquiert simultanément, comme dans le mode de réalisation de la figure 3, une vue de face (image directe I_{d}) et un ensemble de vues de biais (images réfléchies Iᵣ). Bien entendu, les images réfléchies sont inversées par rapport à la réalité. Par exemple, sur l'image réfléchie qui se trouve au-dessus de l'image directe I_{d}, l'arcade supérieure est représentée sous l'arcade inférieure.

Les images réfléchies peuvent avantageusement correspondre à des conditions d'acquisition dans lesquelles l'axe optique de l'appareil d'acquisition est fortement incliné par rapport au plan sagittal. Or l'utilisation de l'appareil d'acquisition dans ces conditions, en observation directe, c'est-à-dire sans miroir, étant souvent difficile pour le patient.

L'image composée est ensuite transmise au module de traitement 59, par des moyens filaires ou non filaires, par exemple par Wifi ou par Bluetooth.

Le traitement d'images permet en particulier, suivant des méthodes de traitement conventionnelles, d'isoler l'image directe et la ou les images réfléchies. Dans un mode de réalisation, le traitement comporte également une opération d'inversion des images réfléchies et/ou une opération de correction des effets de perspective et/ou une opération de correction des couleurs au moyen de la mire colorimétrique 34.

L'analyse d'une des images directe et réfléchies, de préférence de l'image directe, permet de déterminer les conditions d'acquisition de ladite image, de préférence suivant l'enseignement de PCT/EP2015/074896, incorporé par référence. Les conditions d'acquisition des autres images peuvent avantageusement en être déduites, en tenant simplement compte de la géométrie du support 12. En particulier, la calibration de l'appareil d'acquisition est la même pour toutes les images directe et réfléchies. En outre, de simples considérations géométriques permettent de déterminer la position et l'orientation de l'appareil d'acquisition dans l'espace qui, en l'absence de miroir, auraient permis à l'appareil d'acquisition d'acquérir une image réfléchie.

Dans un mode de réalisation, le module de traitement 59, de préférence intégré dans l'appareil d'acquisition d'images 19, commande l'acquisition de plusieurs images composées dans des conditions d'acquisition différentes, et en particulier avec des distances focales différentes. Par exemple, des première, deuxième et troisième images composées peuvent être acquises en focalisant successivement l'appareil d'acquisition sur les incisives, sur les prémolaires et sur les molaires. Avantageusement, chaque dent est ainsi représentée de manière nette sur au moins une des images composées.

Dans un mode de réalisation, la prise de plusieurs images successives dans des conditions d'acquisition différentes résulte d'un unique actionnement du déclencheur de l'appareil d'acquisition d'images. En particulier, l'appareil d'acquisition d'images peut être configuré pour prendre successivement plusieurs photos, avec des distances focales différentes, en conséquence d'un unique déclenchement.

Par ailleurs, l'appareil d'acquisition acquiert de préférence une image des dents du patient représentant également les mires colorimétriques et de translucidité.

Le module de traitement 59, par exemple un applicatif de l'appareil d'acquisition, connaissant les propriétés de couleur et de translucidité réelles des mires, corrige alors l'image jusqu'à ce que les représentations desdites mires sur l'image présentent ces propriétés.

Pour que la correction soit précise, la source lumineuse 51 est de préférence réglée de manière qu'à l'instant de l'acquisition de l'image, les mires soient éclairées dans les mêmes conditions que lors de la mesure des propriétés réelles.

Toutes les images acquises par l'appareil d'acquisition sont ainsi avantageusement comparables, quel que soit l'environnement lumineux extérieur au dispositif au moment de leur acquisition.

Comme cela apparaît clairement à présent, un dispositif selon le premier aspect principal de l'invention permet avantageusement une acquisition très rapide de plusieurs images, typiquement en moins d'une minute, sans avoir recours à une personne spécialisée, notamment un dentiste ou un orthodontiste. L'acquisition d'images peut être en particulier effectuée par le patient lui-même ou par un de ses proches, avec un simple téléphone portable, n'importe où, et en particulier en dehors d'un cabinet médical, dentaire ou d'orthodontie.

En outre, si la géométrie du support, et en particulier l'orientation et le positionnement d'un miroir, est connue, il suffit de déterminer les conditions d'acquisition de l'image directe pour pouvoir déterminer, par simple calcul, les conditions d'acquisition de l'image réfléchie par ce miroir. La mise en oeuvre du procédé décrit dans PCT/EP2015/074896 en est considérablement accélérée.

Enfin, l'appareil d'acquisition 19 étant immobilisé par rapport aux dents, l'image composée est avantageusement nette et, si le dispositif comporte une source lumineuse contrôlée, bien contrastée.

Le dispositif selon le deuxième aspect principal de l'invention permet un suivi de l'évolution des propriétés d'aspect des dents. Il permet également une mesure précise de ces propriétés à tout instant, par le patient lui-même, et en particulier peu avant la réalisation d'une prothèse par exemple. La prothèse présente ainsi un aspect particulièrement proche de celui des dents du patient.

Bien entendu, l'invention n'est pas limitée aux modes de réalisation décrits et représentés, fournis à des fins illustratives seulement.

En particulier, le miroir n'est pas nécessairement plan. Il peut être en particulier configuré pour compenser des effets de perspective et/ou pour refléter des régions particulières de la bouche.

Le miroir peut être mobile par rapport au support, et en particulier peut être mobile en translation, notamment pour autoriser une modification de la distance entre le miroir d'une part et l'ouverture d'écarteur et/ou l'ouverture d'acquisition d'autre part.

Le miroir peut être également mobile en rotation, en particulier autour d'un axe de rotation perpendiculaire à l'axe X de l'écarteur, deux axes étant dits perpendiculaires lorsque deux plans orthogonaux à ces axes sont perpendiculaires l'un à l'autre.

De préférence, l'appareil d'acquisition d'images comporte un applicatif configuré pour guider l'opérateur afin qu'il positionne et oriente correctement le miroir. Dans un mode de réalisation, le positionnement et/ou l'orientation du miroir sont facilités par une échelle portant des indications relatives à différents appareils d'acquisition. L'opérateur peut ainsi facilement disposer le miroir en fonction de l'appareil d'acquisition utilisé.

Dans un mode de réalisation, le dispositif comporte un ou plusieurs actionneurs adaptés pour régler la longueur du support et/ou le positionnement et/ou l'orientation du miroir en fonction de consignes, de préférence en fonction de consignes reçues de l'appareil d'acquisition d'images 19.

Dans un mode de réalisation, le dispositif comporte également un ou plusieurs capteurs configurés pour mesurer la longueur du support 12 et/ou le positionnement et/ou l'orientation du miroir, et un émetteur capable de transmettre ladite mesure à l'appareil d'acquisition d'images. Avantageusement, une configuration optimale du dispositif peut être obtenue par une commande des actionneurs suivant une consigne, de préférence fournie par l'appareil d'acquisition d'images, ladite consigne étant de préférence fonction de l'image observée par l'appareil d'acquisition d'images et/ou par les mesures prises par les capteurs.

Enfin, le nombre de miroirs n'est pas limité et plusieurs miroirs, de préférence tous les miroirs présentent de préférence une ou plusieurs des caractéristiques préférées du miroir 16 décrit précédemment. En particulier, un ou plusieurs des miroirs peuvent être pourvus d'un capteur, d'un actionneur et de moyens de communication avec l'appareil d'acquisition d'images, comme décrit précédemment.

## Revendications

1. Dispositif de prise de vue comportant :
- un support (12) ;
- un écarteur dentaire (14) fixé de manière désactivable sur le support et définissant une ouverture d'écarteur (24) ;
- une mire colorimétrique (52) et/ou une mire de translucidité (54) ; et
- une source lumineuse (51) orientée de manière à éclairer d'une part les dents du patient à travers l'ouverture d'écarteur et d'autre part ladite mire colorimétrique et/ou ladite mire de translucidité ;
- des moyens de fixation (18) d'un appareil d'acquisition d'images (19) sur le support dans une position dans laquelle l'appareil d'acquisition est orienté pour recevoir une image (I_{c}) de l'ouverture d'écarteur d'une part et de ladite mire colorimétrique et/ou de ladite mire de translucidité d'autre part, lesdits moyens de fixation étant désactivables.

2. Dispositif selon la revendication immédiatement précédente, comportant un module de contrôle (60) configuré pour contrôler les propriétés du rayonnement émis par la source lumineuse (51).

3. Dispositif selon la revendication immédiatement précédente, dans lequel le module de contrôle (60) est configuré pour contrôler les propriétés du rayonnement émis par la source lumineuse (51) en fonction du rayonnement lumineux reçu par l'ouverture d'écarteur.

4. Dispositif selon la revendication immédiatement précédente, dans lequel le module de contrôle (60) est configuré pour commander la source lumineuse (51) pour que plus de 50%, plus de 70%, plus de 90%, voire sensiblement 100% de l'intensité du rayonnement reçu par l'ouverture d'écarteur proviennent de la source lumineuse (51).

5. Dispositif selon l'une quelconque des revendications précédentes, dans lequel le support présente la forme d'un boîtier ne débouchant vers l'extérieur sensiblement que par l'ouverture d'écarteur et par une ouverture d'acquisition (26) à travers laquelle l'appareil d'acquisition fixé par lesdits moyens de fixation (18) reçoit ladite image (I_{c}).

6. Dispositif selon l'une quelconque des revendications précédentes, dans lequel l'écarteur est fixé de manière amovible sur le support.

7. Dispositif selon la revendication immédiatement précédente, dans lequel l'écarteur comporte des pattes insérées dans des logements profilés respectifs présentant chacun une section transversale de forme générale en U.

8. Dispositif selon la revendication immédiatement précédente, dans lequel l'ouverture du U débouche vers le haut ou vers le bas dans une position de service dans laquelle l'écarteur est disposé sur la bouche d'un patient maintenant sa tête verticale.

9. Dispositif selon l'une quelconque des revendications précédentes, dans lequel l'écarteur est fixé sur le support dans une position montée dans laquelle il est maintenu en flexion.

10. Dispositif selon l'une quelconque des revendications précédentes, comportant un module de traitement (59) dans lequel sont enregistrées les propriétés de couleur et de translucidité réelles des mires colorimétrique et de translucidité, respectivement, le module de traitement comprenant des instructions de code de programme pour corriger l'image jusqu'à ce que les représentations desdites mires sur l'image présentent lesdites propriétés.
